# EUROPEAN PATENT SPECIFICATION

(11) **EP 2 918 304 B1**
(45) Date of publication and mention of the grant of the patent: **07.08.2019**
(21) Application number: 15154085.3
(22) Date of filing: 06.02.2015
(51) Int. Cl.: A61M 25/00, A61M 25/10

(54) **Balloon catheter**
Ballonkatheter
Cathéter de Fogarty

(30) Priority: 20.02.2014 JP 2014030221
(43) Date of publication of application: 16.09.2015
(73) Proprietor: Asahi Intecc Co., Ltd., Seto-shi, Aichi 489-0071 (JP)
(72) Inventor: Kubo, Yuta, Seto-shi, Aichi 489-0071 (JP); Ikegaya, Michihiro, Seto-shi, Aichi 489-0071 (JP); Kaneko, Yoshiki, Seto-shi, Aichi 489-0071 (JP)
(74) Representative: Winter, Brandl, Fürniss, Hübner, Röss, Kaiser, Polte - Partnerschaft mbB

(56) References cited:
- US-A- 6 010 521
- US-A1- 2002 082 550
- US-A1- 2006 142 733
- US-A1- 2014 025 045

## Description

### [Technical Field]

The present invention relates to a balloon catheter inserted into a stenosis formed in a blood vessel such that the catheter enlarges the stenosis to obtain a blood flow.

### [Background Art]

Conventionally, balloon catheters are known as therapeutic catheters that are inserted into stenoses formed in blood vessels and are enlarged therein. A balloon catheter mainly includes a balloon acting as an inflating body, an outer shaft welded to the rear end of the balloon, and an inner shaft inserted into the balloon and the outer shaft. The inner shaft is used for inserting a guide wire. An inflation lumen provided between the outer shaft and the inner shaft is used for passing a liquid (e.g., a contrast medium and a physiological saline) for inflating the balloon.

The end of the inner shaft has a front-end tip made of soft resin. Thus, even if an operator presses the balloon catheter in a distal direction so as to hit the front end of the balloon catheter to a blood vessel wall, the balloon catheter hardly breaks the blood vessel wall.

When the balloon catheter configured thus is inserted into a stenosis, the soft front-end tip may be caught by the stenosis . If the balloon catheter is forcibly rotated or pulled by an operator with the front-end tip caught by the stenosis, unfortunately, the front-end tip cannot be removed from the stenosis and may be broken from the inner shaft. As a solution to this problem, a known balloon catheter includes a connecting tube that covers the front end of an inner shaft and the rear end of a front-end tip from the outside (For example, see Patent Literature 1).

In the balloon catheter of Patent Literature 1, however, when a front-end tip having a short length is used, a welding area between the outer surface of the front-end tip and the inner surface of the connecting tube is small. Unfortunately, this reduces the welding strength between the front-end tip and the connecting tube and thus the front-end tip caught by the stenosis still may be broken from the front end of the inner shaft.

### [Citation List]

### [Patent Literature]

[Patent Literature 1] US 6,918,920

US 2002/0082550 A1 describes a balloon catheter with soft distal tip having a balloon on the distal shaft section and a tip member on a distal end of the catheter having a proximal end spaced distally apart from the distal end of the shaft.

US 6010521 A describes a balloon catheter having a fusion bonded distal tip with an outer layer covering the distal tip.

### [Problem to be Solved by the Invention]

The present invention has been devised in view of these circumstances . An object of the present invention is to provide a balloon catheter in which a thick portion formed by a connecting tube embedded into a front-end tip increases an anchor effect and a welding area between the outer surface of the front-end tip and the inner surface of the connecting tube. Thus, even if the front-end tip is caught by a stenosis, the front-end tip is hardly broken from the front end of an inner shaft.

### [Solution to Problem]

The problem is solved by the following solutions:

A first aspect of the present invention is a balloon catheter including: a balloon, an inner shaft welded to a front end of the balloon, a front-end tip welded to the front end of the inner shaft, and a connecting tube covering an outer surface on the front end of the inner shaft and the rear end of the front-end tip, wherein the connecting tube has a thick portion that is embedded into the front-end tip.

A second aspect of the present invention is the balloon catheter according to the first aspect, wherein the connecting tube is made of resin having higher stiffness than the front-end tip and lower stiffness than the inner shaft.

A third aspect of the present invention is the balloon catheter according to the first or second aspect, wherein the thick portion is in contact with the front end of the inner shaft.

A fourth aspect of the present invention is the balloon catheter according to any one of the first to third aspects, wherein the connecting tube has a rear end that covers the front end of the balloon.

### [Advantageous Effects of Invention]

In the balloon catheter according to the first aspect of the present invention, the connecting tube has a thick portion that is embedded into the front-end tip. Thus, even if the front-end tip is caught by a stenosis, by the anchor effect of the thick portion of the connecting tube, the occurrence of breaks on the front-end tip from the inner shaft can be further reduced. Moreover, the connecting tube having the thick portion can increase a welding area between the outer surface of the front-end tip and the inner surface of the connecting tube. Even if the front-end tip has a short length, the occurrence of breaks on the front-end tip from the inner shaft can be reduced.

In the balloon catheter according to the second aspect of the present invention, the connecting tube is made of resin having higher stiffness than the front-end tip and lower stiffness than the inner shaft. This can reduce a difference in stiffness between the front end of the inner shaft and the rear end of the front-end tip. Thus, even if the balloon catheter is inserted into a curved blood vessel, a tensile stress caused by the curved blood vessel hardly concentrates between the front end of the inner shaft and the rear end of the front-end tip. Moreover, the thick portion of the connecting tube having medium stiffness suppresses a tensile stress generated in a distal direction. This can reduce the occurrence of breaks on the front-end tip from the front end of the inner shaft.

In the balloon catheter according to the third aspect of the present invention, the thick portion of the connecting tube is in contact with the front end of the inner shaft. Thus, a pressing force applied by an operator in the distal direction of the balloon catheter can be gradually transmitted from the inner shaft to the front-end tip through the thick portion of the connecting tube. This can improve the pressing force of the balloon catheter.

In the balloon catheter according to the fourth aspect of the present invention, the rear end of the connecting tube extends to and covers the front end of the balloon. This can reduce the possibility of removal of the balloon from the inner shaft even if a high pressure is applied to the balloon.

### [Brief Description of Drawings]

FIG. 1 is an overall view of a balloon catheter according to the present embodiment.
FIG. 2(A) is an enlarged view of part A of FIG. 1, FIG. 2 (B) is a cross-sectional view taken along line C-C of FIG. 2(A), FIG. 2 (C) is a cross-sectional view taken along line D-D of FIG. 2(A), and FIG. 2(D) is a modification of FIG. 2(B).
FIG. 3 is an enlarged view of part B of FIG. 2(A), illustrating a state of the balloon catheter inserted into a curved blood vessel.
FIG. 4 shows a modification of FIG. 2(A), in which the thick portion of a connecting tube is in contact with the front end of an inner shaft.
FIG. 5 shows a modification of FIG. 2(A), in which the rear end of the connecting tube is extended to and covers the front end of the balloon.
FIGS. 6(A) and 6(B) show modifications of FIG. 2 (A) .

### [Description of Embodiments]

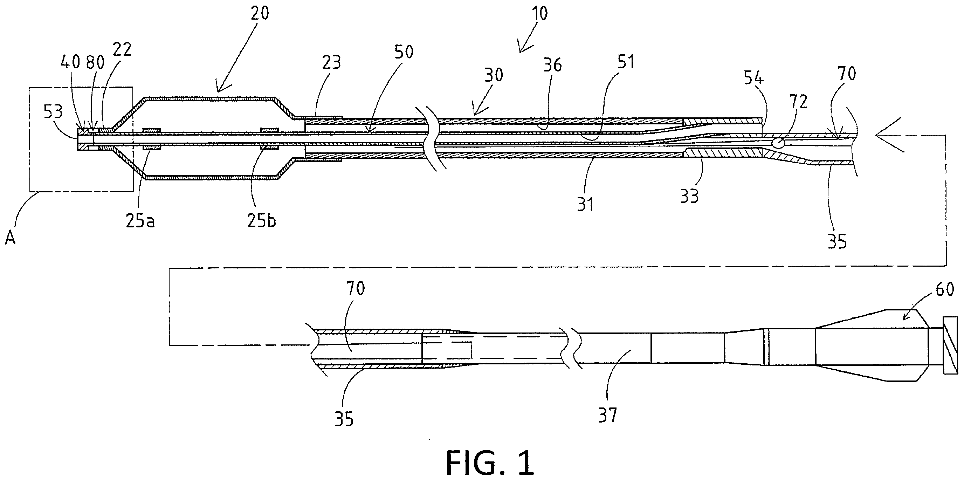

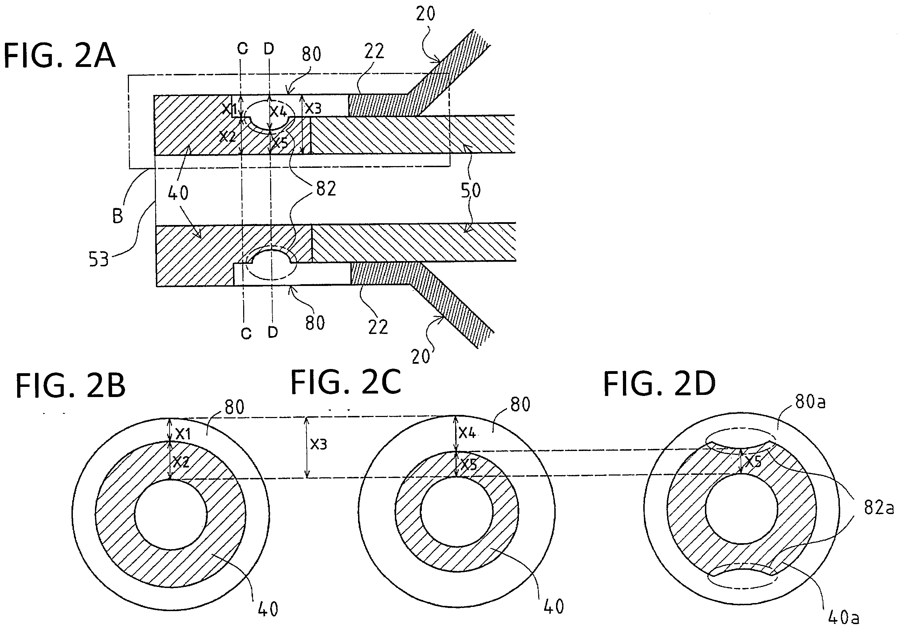

Referring to FIGS. 1 to 2(D), a balloon catheter 10 according to the present embodiment will be described in the following example. In FIGS. 1 and 2(A), the left side indicates a front end (dismal side) to be inserted into a body while the right side indicates a rear end (proximal side) operated by an operator, e.g., a doctor.

For example, the balloon catheter 10 is used for enlarging and treating a stenosis formed in a heart vessel. As shown in FIG. 1, the balloon catheter 10 mainly includes a balloon 20, an outer shaft 30, a front-end tip 40, an inner shaft 50, a connector 60, a reinforcing member 70, and a connecting tube 80.

The balloon 20 for enlarging a stenosis is a resin member including a front-end attachment part 22 on the front end and a rear-end attachment part 23 on the rear end. The front-end attachment part 22 is welded to the front end of the inner shaft 50 and the rear end of the connecting tube 80 while the rear-end attachment part 23 is welded to the front end of the outer shaft 30. In FIG. 1, although the rear-end attachment part 23 is welded to the outer surface of the front end of the outer shaft 30, the present embodiment is not limited to this configuration. The rear-end attachment part 23 may be welded to the inner surface of the front end of the outer shaft 30 instead.

The outer shaft 30 is a cylindrical member constituting an inflation lumen 36 for supplying a liquid such as a contrast medium and a physiological saline to inflate the balloon 20. The outer shaft 30 includes, from the front end, a front-end outer shaft 31, a guide wire port 33, an intermediate outer shaft 35, and a rear-end outer shaft 37. The front-end outer shaft 31 and the intermediate outer shaft 35 are tubes made of resins such as polyamide, polyamide elastomer, polyolefin, polyester, and polyester elastomer. The guide wire port 33 is a welded part of the front-end outer shaft 31, the intermediate outer shaft 35, and the inner shaft 50.

The inner shaft 50 is inserted into the front-end outer shaft 31. The inflation lumen 36 is formed between the front-end outer shaft 31 and the inner shaft 50.

The rear-end outer shaft 37 is a metallic cylindrical member that is called a hypotube. The front end of the rear-end outer shaft 37 is inserted into the rear end of the intermediate outer shaft 35 and is welded therein. The connector 60 is attached to the rear end of the rear-end outer shaft 37. When a liquid such as a contrast medium and a physiological saline is supplied to inflate the balloon 20 from an indeflator (not shown) attachable to the connector 60, the liquid passes through the inflation lumen 36 and inflates the balloon 20. The material of the rear-end outer shaft 37 is not particularly limited. The rear-end outer shaft 37 may be made of a superelastic alloy such as stainless steel (SUS304) and a Ni-Ti alloy.

The inner shaft 50 forms a guide wire lumen 51 in which a guide wire is inserted. The rear end of the inner shaft 50 is joined to the guide wire port 33 of the outer shaft 30 to form a rear-end guide wire port 54.

As will be described later, the front-end tip 40 is welded to the front end of the inner shaft 50. The front-end tip 40 is made of soft resin. The material is not particularly limited and materials such as polyurethane and polyurethane elastomer may be used. The front-end tip 40 has a front-end guide wire port 53 on the front end.

The inner shaft 50 includes radio-opaque markers 25a and 25b that are attached in the balloon 20 to locate the balloon 20 under radiation exposure. The number and locations of the markers 25a and 25b can be optionally changed according to the length of the balloon 20.

The reinforcing member 70 is attached to the inner surface of the front end of the rear-end outer shaft 37. The reinforcing member 70 is circular in cross section and is a tapered metallic wire rod that decreases in diameter toward the end of the reinforcing member 70. The material of the reinforcing member 70 is not particularly limited. The reinforcing member 70 may be made of a superelastic alloy such as stainless steel (SUS304) and a Ni-Ti alloy. The reinforcing member 70 passes through the intermediate outer shaft 35 and the guide wire port 33 and then extends to the front-end outer shaft 31.

The reinforcement 70 has a pressing part 72 nearly between the intermediate outer shaft 35 and the guide wire port 33. When an operator presses the balloon catheter 10 in a distal direction, the pressing part 72 comes into contact with the guide wire port 33 so as to transmit a pressing force of the operator from the guide wire port 33 to the outer shaft 30 and the inner shaft 50. The pressing part 72 is preferably made of the same material as the reinforcement 70.

FIG. 2(A) is an enlarged view of part A of FIG. 1. The front-end attachment part 22 of the balloon 20 is welded to the front end of the inner shaft 50. The connecting tube 80 covers the front end of the inner shaft 50 and the rear end of the front-end tip 40 from the outside. The connecting tube 80 has a thick portion 82 embedded into the front-end tip 40.

FIG. 2 (B) is a cross-sectional view taken along line C-C of FIG. 2(A). FIG. 2(C) is a cross-sectional view taken along line D-D of FIG. 2(A). X1 denotes the thickness of the connecting tube 80 other than the thick portion 82, X2 denotes the thickness of the front-end tip 40 other than the thick portion 82 (See FIG. 2(B)), X4 denotes the thickness of the connecting tube 80 on the thick portion 82, X5 denotes the thickness of the front-end tip 40 on the thick portion 82 (See FIG. 2(C)), and X3 denotes the sum of the thickness of the connecting tube 80 and the thickness of the front-end tip 40. The sum X3 of the thickness of the connecting tube 80 and the thickness of the front-end tip 40 is equal in the longitudinal direction (In other words, X1 + X2 = X4 + X5 = X3) on a portion other than the thick portion 82 (See FIG. 2(B)) and on the thick portion 82 (See FIG. 2(C)). Thus, on the thick portion 82, the front-end tip 40 has a smaller thickness (X5 < X2) and the connecting tube 80 has a larger thickness (X4 > X1) than on a portion other than the thick portion 82.

The thick portion 82 of the connecting tube 80 is embedded into the front-end tip 40. Thus, even if the front-end tip 40 is caught by a stenosis, by the anchor effect of the thick portion 82 of the connecting tube 80, the occurrence of breaks on the front-end tip 40 from the front end of the inner shaft 50 can be reduced. Moreover, the thick portion 82 of the connecting tube 80 can increase a welding area between the outer surface of the front-end tip 40 and the inner surface of the connecting tube 80. Even if the front-end tip 40 has a short length, the occurrence of breaks on the front-end tip 40 from the front end of the inner shaft 50 can be reduced.

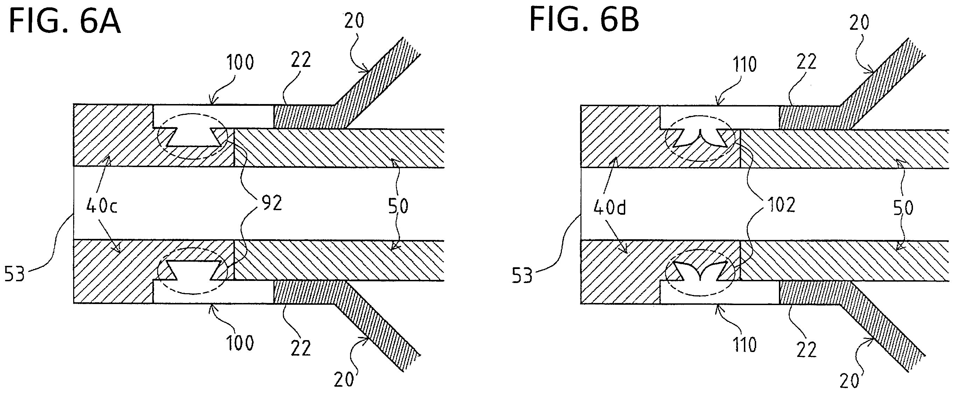

In FIGS. 2(A) and 2(C), the thick portion 82 of the connecting tube 80 is provided over the entire circumference (360°) of the connecting tube 80. The present embodiment is not limited to this configuration. For example, as shown in FIG. 2(D), thick portions 82a may be provided on a certain portion of the connecting tube 80a. In FIG. 2(D), two thick portions 82a are embedded into a front-end tip 40a. The number of thick portions 82a may be optionally adjusted. Moreover, the shapes of the thick portions 82 and 82a are not particularly limited and thus may be shaped like thick portions 92 and 102 shown in FIGS. 6(A) and 6(B). As shown in FIG. 6(A), a thick portion 92 of a connecting tube 100 is embedded into a front-end tip 40c or as shown in FIG. 6(B), a thick portion 102 of a connecting tube 110 is embedded into a front-end tip 40d. Thus, even if the front-end tips 40c and 40d are caught by stenosis, by an anchor effect, the occurrence of breaks on the front-end tips 40c and 40d from the front end of the inner shaft 50 can be further reduced.

In the above explanation, the thick portions 82, 82a, 92, and 102 are longitudinally embedded in the front-end tips 40, 40a, 40c, and 40d, respectively. Each of the thick portions 82, 82a, 92, and 102 may be replaced with multiple thick portions longitudinally provided on each of the front-end tips. The provision of the multiple thick portions 82, 82a, 92, 102 in the longitudinal direction can increase the anchor effect and the welding areas between the front-end tips 40, 40a, 40c, and 40d and the connecting tubes 80, 80a, 100, and 110, thereby preventing the front-end tips 40, 40a, 40c, and 40d from being broken from the front end of the inner shaft 50.

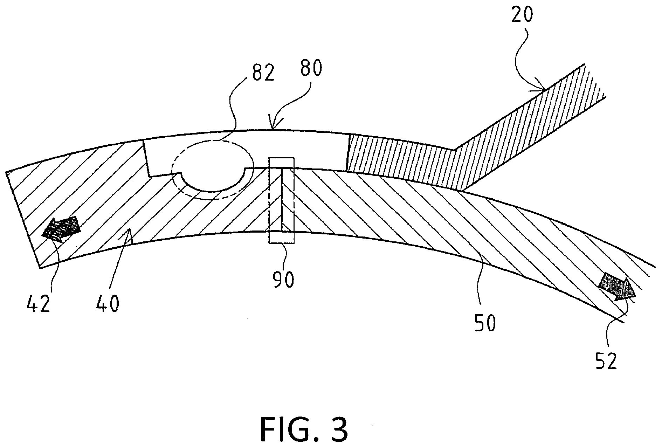

FIG. 3 shows the balloon catheter 10 inserted into a curved blood vessel. For illustration, FIG. 3 only shows an enlarged view of part B of FIG. 2 (A) .

When the balloon catheter 10 is inserted into the curved blood vessel, the front-end tip 40 is pulled to the front end by a tensile stress 42 applied along the curved blood vessel; meanwhile, the inner shaft 50 is pulled to the rear end by a tensile stress 52 applied along the curved blood vessel. Thus, a difference in stiffness on a boundary 90 between the rear end of the front-end tip 40 and the front end of the inner shaft 50 may cause stress concentration on the boundary 90. This may break the front-end tip 40 from the front end of the inner shaft 50. If soft resin is used for the inner shaft 50 as the front-end tip 40 to reduce a difference in stiffness on the boundary 90 between the rear end of the front-end tip 40 and the front end of the inner shaft 50, it is difficult to transmit a pressing force of an operator to the front-end tip 40, leading to difficulty in inserting the balloon catheter into a stenosis.

Hence, in the balloon catheter 10, the connecting tube 80 covering an outer surface on the rear end of the front-end tip 40 and the front end of the inner shaft 50 is made of resin having higher stiffness than the front-end tip 40 and lower stiffness than the inner shaft 50. The inner shaft 50 made of resin having high stiffness allows efficient transmission of a pressing force of an operator to the front-end tip 40 through the inner shaft 50. Furthermore, the connecting tube 80 made of resin having medium stiffness can reduce a difference in stiffness on the boundary 90 between the rear end of the front-end tip 40 and the front end of the inner shaft 50. Thus, the balloon catheter 10 inserted into the curved blood vessel does not cause stress concentration on the boundary 90 between the rear end of the front-end tip 40 and the front end of the inner shaft 50, thereby reducing the occurrence of breaks on the front-end tip 40 from the front end of the inner shaft 50. The thick portion 82 of the connecting tube 80 having medium stiffness can suppress the tensile stress 42 generated in the distal direction during curving, thereby further reducing the occurrence of breaks on the front-end tip 40 from the front end of the inner shaft 50.

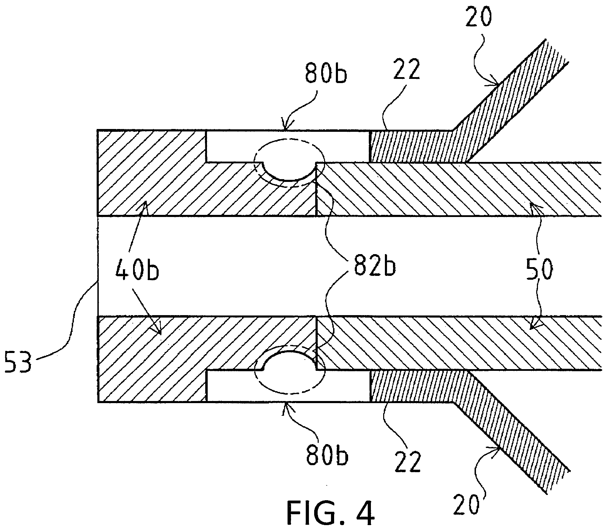

FIG. 4 shows that a thick portion 82b of a connecting tube 80b is in contact with the front end of the inner shaft 50. The thick portion 82b in contact with the front end of the inner shaft 50 can gradually transmit a pressing force applied by an operator in the distal direction of the balloon catheter 10, from the inner shaft 50 to the front-end tip 40b through the thick portion 82b of the connecting tube 80b. If the inner shaft 50 is made of resin having high stiffness, the front-end tip 40b is made of resin having low stiffness, and the connecting tube 80b is made of resin having higher stiffness than the front-end tip 40b and lower stiffness than the inner shaft 50, in particular, the thick portion 82b having medium stiffness is in contact with the front end of the inner shaft 50, thereby reducing a loss of a pressing force on the boundary 90 between the front end of the inner shaft 50 and the rear end of the front-end tip 40b. This can improve the operability of the balloon catheter 10 for an operator.

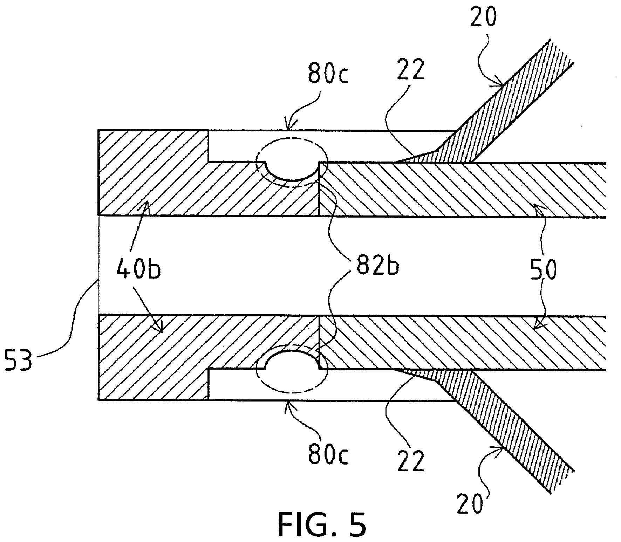

FIG. 5 shows that the rear end of a connecting tube 80c is extended to the front-end attachment part 22 of the balloon 20 so as to cover the front end of the inner shaft 50 and the front-end attachment part 22 of the balloon 20. The connecting tube 80c covering the front-end attachment part 22 of the balloon 20 can reduce the possibility of removal of the balloon 20 from the inner shaft 50 even if a liquid with a high pressure passes through the balloon 20.

The shapes of the front-end tips 40, 40a, 40b, 40c, and 40d are not limited to those of FIGS. 1 to 6(B). The front-end tip may be tapered with an outside diameter decreasing toward the front end. In FIGS. 1 to 6(B), for simplification, the connecting tubes 80, 80a, 80b, 80c, 100, and 110 have an equal thickness except for the thick portions 82, 82a, 82b, 92, and 102. However, if the connecting tubes 80, 80a, 80b, 80c, 100, and 110 are made of resin having higher stiffness than the front-end tips 40, 40a, 40b, 40c, and 40d and lower stiffness than the inner shaft 50, the connecting tubes 80, 80a, 80b, 80c, 100, and 110 covering the outer surface of the inner shaft 50 are preferably reduced in thickness to further reduce a difference in stiffness between the rear ends of the front-end tips 40, 40a, 40b, 40c, and 40d and the front end of the inner shaft 50; meanwhile, the connecting tubes 80, 80a, 80b, 80c, 100, and 110 covering the outer surfaces of the front-end tips 40, 40a, 40b, 40c, and 40d are preferably increased in thickness. In other words, the connecting tubes 80, 80a, 80b, 80c, 100, and 110 are preferably increased in thickness from the rear ends to the front ends except for the thick portions 82, 82a, 82b, 92, and 102.

In the balloon catheter 10, the thick portions 82, 82a, 82b, 92, and 102 of the connecting tubes 80, 80a, 80b, 80c, 100, and 110 are embedded into the front-end tips 40, 40a, 40b, 40c, and 40d. This increases the anchor effect of the thick portions 82, 82a, 82b, 92, and 102 of the connecting tubes 80, 80a, 80b, 80c, 100, and 110 and welding areas between the outer surfaces of the front-end tip 40, 40a, 40b, 40c, and 40d and the inner surfaces of the connecting tubes 80, 80a, 80b, 80c, 100, and 110, thereby reducing the occurrence of breaks on the front-end tips 40, 40a, 40b, 40c, and 40d from the front end of the inner shaft 50.

### [Reference Signs List]

- 10: balloon catheter
- 20: balloon
- 22: front-end attachment part
- 23: rear-end attachment part
- 25a, 25b: marker
- 30: outer shaft
- 31: front-end outer shaft
- 33: guide wire port
- 35: intermediate outer shaft
- 36: inflation lumen
- 37: rear-end outer shaft
- 40, 40a, 40b, 40c, 40d: front-end tip
- 42, 52: tensile stress
- 50: inner shaft
- 51: guide wire lumen
- 53: front-end guide wire port
- 54: rear-end guide wire port
- 60: connector
- 70: reinforcing member
- 72: pressing part
- 80, 80a, 80b, 80c, 100, 110: connecting tube
- 82, 82a, 82b, 92, 102: thick portion
- 90: boundary

## Claims

1. A balloon catheter (10) comprising:
a balloon (20);
an inner shaft (50) welded to a front end of the balloon;
a front-end tip (40, 40a, 40b, 40c, 40d) welded to a front end of the inner shaft; and
a connecting tube (80, 80a, 80b, 80c, 100, 110) covering: an outer surface on the front end of the inner shaft and a rear end of the front-end tip,
**characterized in that**:
the connecting tube has a thick portion (82, 82a, 82b, 92, 102) that is embedded into the front-end tip.

2. The balloon catheter according to claim 1, wherein the connecting tube (80, 80a, 80b, 80c, 100, 110) is made of resin having higher stiffness than the front-end tip and lower stiffness than the inner shaft.

3. The balloon catheter according to claim 1 or 2, wherein the thick portion (82b) is in contact with the front end of the inner shaft.

4. The balloon catheter according to any one of claims 1 to 3, wherein the connecting tube (80c) has a rear end that covers the front end of the balloon.

## Patentansprüche

1. Ballonkatheter (10) mit:
einem Ballon (20);
einem an ein vorderes Ende des Ballons geschweißten Innenschaft (50);
einer an ein vorderes Ende des Innenschafts geschweißten vorderen Spitze (40, 40a, 40b, 40c, 40d); und
einem Verbindungsrohr (80, 80a, 80b, 80c, 100, 110), das das vordere Ende des Innenschafts und ein hinteres Ende der vorderen Spitze außenseitig ummantelt, **dadurch gekennzeichnet, dass**:
das Verbindungsrohr eine Verdickung (82, 82a, 82b, 92, 102) hat, die in der vorderen Spitze eingebettet ist.

2. Ballonkatheter nach Anspruch 1, wobei das Verbindungsrohr (80, 80a, 80b, 80c, 100, 110) aus Harz hergestellt ist, das eine höhere Steifigkeit als die vordere Spitze und eine geringere Steifigkeit als der Innenschaft hat.

3. Ballonkatheter nach Anspruch 1 oder 2, wobei die Verdickung (82b) mit dem vorderen Ende des Innenschafts in Kontakt ist.

4. Ballonkatheter nach einem der Ansprüche 1 bis 3, wobei ein hinteres Ende des Verbindungsrohrs (80c) das vordere Ende des Ballons ummantelt.

## Revendications

1. Cathéter à ballonnet (10) comprenant :
un ballonnet (20) ;
une tige interne (50) soudée à une extrémité antérieure du ballonnet ;
un bout d'extrémité antérieure (40, 40a, 40b, 40c, 40d) soudé à une extrémité antérieure de la tige interne ; et
un tube de liaison (80, 80a, 80b, 80c, 100, 110) couvrant une surface externe sur l'extrémité antérieure de la tige interne et une extrémité postérieure du bout d'extrémité antérieure,
**caractérisé en ce que** :
le tube de liaison a une partie épaisse (82, 82a, 82b, 92, 102) qui est incluse dans le bout d'extrémité antérieure.

2. Cathéter à ballonnet selon la revendication 1, où le tube de liaison (80, 80a, 80b, 80c, 100, 110) est fait d'une résine ayant une plus grande rigidité que le bout d'extrémité antérieure et une petite rigidité que la tige interne.

3. Cathéter à ballonnet selon la revendication 1 ou 2, où la partie épaisse (82b) est en contact avec l'extrémité antérieure de la tige interne.

4. Cathéter à ballonnet selon l'une quelconque des revendications 1 à 3, où le tube de liaison (80c) a une extrémité postérieure qui couvre l'extrémité antérieure du ballonnet.
